# EUROPEAN PATENT APPLICATION

(11) **EP 4 671 384 A1**
(43) Date of publication of application: **31.12.2025**
(21) Application number: 25184953.5
(22) Date of filing: 24.06.2025
(51) Int. Cl.: C12Q 1/6883

(54) **HSA-MIR-106B-5P AND HSA-MIR-15A-5P AS BIOMARKERS OF ANTIEPILEPTIC DRUG RESPONSE IN PATIENTS WITH EPILEPSY**

(30) Priority: 26.06.2024 IT 202400014710
(71) Applicant: Genechron S.r.l., 00143 Roma (IT)
(72) Inventor: SPEDALETTI, Valentina, I-00054 FIUMICINO (Roma) (IT); ROSATO, Vittorio Camillo, I-00142 ROMA (IT); ANGELUCCI, Emanuela, I-00153 ROMA (IT)
(74) Representative: Comoglio, Elena

(57) **Abstract**

The invention concerns an *in vitro* method for assessing the epileptic condition of a patient. The method comprises determining in a serum sample from said patient the level of a mi-croRNA selected from hsa-miR-106b-5p and hsa-miR-15a-5p, said level being indicative of the epileptic seizure frequency in the patient.

## Description

The present invention falls within the field of diagnostics. In particular, it relates to an *in vitro* method for assessing the seizure frequency and response to anti-epileptic drugs of patients with epilepsy.

The most common long-term neurological condition affecting children is epilepsy, which has a variety of aetiologies, including genetic, structural, metabolic, infectious or immunological. In some cases, the cause of epilepsy is still unknown. With the appropriate drugs, about 70% of people with active epilepsy can experience long-term seizure remission. More than thirty anti-epileptic drugs (ASMs) are available; however, about 30% of epilepsy patients experience seizures that are resistant to specific drug classes and are unable to achieve seizure independence with inappropriate drug therapy. The variability in response to ASMs therefore represents a challenge for effective epilepsy management.

Currently, it is not possible to reliably predict how the individual patient will respond to drug treatment, which highlights the need to identify effective biomarkers useful in this regard.

It should also be considered that, although the intensity and frequency of a patient's seizures is evidently correlated with his response to the therapy administered, sometimes or even frequently, seizures occur in such a mild form that they are not easily perceptible on simple observation. This makes it impossible to detect the occurrence of seizures, and therefore to quantify their frequency over time, except by means of instrumental tests such as electroencephalography (EEG). However, such examinations are complex to carry out, especially for paediatric patients, and in any case do not allow constant monitoring over time but are limited only to the time the examination is performed.

In the light of the above, it is apparent that the identification of biomarkers whose levels in serum correlate with the frequency of epileptic seizures and are therefore indicative of the response to the drug therapy administered could be extremely beneficial, significantly improving the management of epilepsy and reducing the burden on both patients and the National Health System.

MicroRNAs (miRNAs) are a family of small non-coding RNAs that influence the post-transcriptional expression of genes. The expression levels of miRNAs thus reflect the functions of the cell, given their function as fine regulators of gene expression. When miRNAs are functionally associated with the process of interest, it is possible to identify the temporal-spatial specificity of their expression, generating a specific expression pattern for each organ, tissue or cell type of interest. It has also been found that miRNAs are stable in serum, making the determination of miRNA levels in blood widely accessible, rapid, painless and convenient.

Recently, several genome-wide miRNA expression targeting and profiling studies have revealed more than one hundred different miRNAs in animal models or epilepsy patients, providing strong evidence that epilepsy is linked to extensive changes in miRNA expression. While several circulating miRNAs have been linked to epilepsy, further research is needed to determine whether these miRNAs are accurate in diagnosing or predicting the risk of epilepsy.

miR-106b-5p has been reported to be upregulated in patients with epilepsy (An et al. 2016). Furthermore, the cited study demonstrated the highest diagnostic accuracy and improved sensitivity and specificity for epilepsy prediction when combined with miR-146a. Toll-like receptors (TLRs) and mediators of the downstream cytokine pathway, including interleukin-1 receptor-associated kinase 1 (IRAK1) and tumour necrosis factor receptor-associated factor 6 (TRAF6), are targets of miR-146a-5p, which is known to be an important regulator of the immune system. Although dysregulation of miR-146a-5p has reasonable diagnostic potential to differentiate epileptic patients from healthy controls, the most recent investigations did not detect any substantial dysregulation in patients who had resistant epilepsy and thus were no longer responding to medication (Organista-Juárez et al.2019).

Sun et al. (2016) demonstrated that miR-106b and miR-15a levels are higher at seizure onset than post-convulsions levels in the serum of patients with epilepsy. However, in this study, miR-106b and miR-15a expression levels were not found to be associated with age, gender, years post diagnosis or seizure frequency.

With regard to the determination of drug-resistant epilepsy, WO2016139599 describes a method based on the identification of the metabolites 3-OH-butyrate, acetoacetate, choline, alanine, glutamate, scyllo-inositol, glucose, lactate and citrate in a plasma sample. However, this method, requiring the determination of all the above metabolites in order to construct a metabolic profile, is rather complex and expensive.

The research carried out by the present inventors therefore aimed to overcome the limitations of the prior art.

With their research, the inventors have now identified two miRNAs that have independently proven to be suitable for use as markers indicative of response to anti-epileptic drugs, particularly in a paediatric population or in subjects not older than about 20 years. These miRNAs are microRNA-106b-5p (hsa-miR-106b-5p) and microRNA 15a-5p (hsa-miR 15a-5p). Both molecules are known per se. The sequence of hsa-miR-106b-5p is available in miRBase under the accession number MIMAT0000680 (sequence: UAAAGUGCUGACAGCAGAU, SEQ ID NO:1). The sequence of hsa-miR-15a-5p is available in miRBase under access number MIMAT0000068 (sequence: UAGCAGCACAUAAUGGUUG, SEQ ID NO:2). However, to the best of the inventors' knowledge, a correlation between the serum level of hsa-miR-106b-5p or hsa-miR-15a-5p and seizure frequency, and thus with the assessment of the response to anti-epileptic drugs, had not been described so far.

Therefore, an aspect of the present invention is an *in vitro* method for assessing the epileptic condition of a patient, wherein the method comprises determining, in a serum sample of said patient, the level of a microRNA selected from hsa-miR-106b-5p and hsa-miR-15a-5p, said level being indicative of the frequency of the patient's epileptic seizures.

In a preferred embodiment of the method, the patient is undergoing anti-epileptic drug therapy and therefore the serum level of said microRNA, being correlated with the frequency of the patient's epileptic seizures, is indicative of the patient's response to the anti-epileptic drug therapy to which the patient is subjected. More preferably, in this embodiment, a serum level of said microRNA below a reference value calculated from one or more reference patients responding to therapy is indicative of the patient's failure to respond to anti-epileptic drug therapy.

In the context of the present description, the expression 'failure to respond to the therapy therapy' means a condition of non-responsiveness to ongoing therapy, or reduced responsiveness to the therapy, due to any known or unknown cause. According to a meaning included in the previous one but more specific, the expression "failure to respond to the therapy" indicates a condition of drug resistance.

In the context of this description, 'drug resistance' means epilepsy that does not respond to properly conducted drug therapy (e.g. at least two drugs and appropriate doses) and for an appropriate observation period.

Further features and advantages of the invention are defined in the dependent claims. The claims form an integral part of the present description.

According to a preferred embodiment of the method of the invention, the level of hsa-miR-106b-5p or hsa-miR-15a-5p is determined by reverse transcription-qPCR (RT-qPCR).

The results obtained by the present inventors, described in detail in the experimental section below, show that the quantification of serum levels of hsa-miR-106b-5p or hsa-miR-15a-5p allows discrimination between patients who have a high frequency of seizures (daily, weekly and monthly) versus patients who have low seizure frequencies (sporadic) or who are seizure-free. These results indicate the possibility of the aforementioned miRNAs for the assessment, over time, of a patient's epileptic condition and his or her actual response (or non-response) to the therapies carried out. These results were also confirmed by a longitudinal study of 47 of the patients previously analysed at t=0 and re-analysed under the same experimental conditions at t=1 (after 12 months). The patients were divided into two subgroups: improved (without seizures and with sporadic seizures) and stable-worsened (with daily, weekly and monthly seizures). The aim of this further evaluation was to check the persistence of the correlation between the markers analysed and the patient's clinical situation (in particular correlation with seizure frequency).

The analysis showed that miR-15a-5p and miR-106b-5p show significantly greater changes in patients who improve in seizure frequency.

Surprisingly, no relationship was found between seizure frequency and serum level of miR-146a-5p, although in the prior art miR-146a-5p had been related to drug resistance. Also in the longitudinal study of the 47 patients previously analysed at t=0 and re-analysed at t=1 (after 12 months) miR-146a-5p showed no significant differences between the two subgroups, improved and stable-worsened.

It is also important to emphasise that, advantageously, the correlation identified by the inventors between hsa-miR-106b-5p or hsa-miR-15a-5p levels and seizure frequency is independent of the state of haemolysis of the blood sample taken, haemolysis which is frequently observed in clinical practice, especially among children. This means that the aforementioned miRNAs can easily be used as clinically useful biomarkers by carrying out a normal blood collection without the need for special precautions.

The research performed by the present inventors and the results obtained are described in the experimental section below, which is provided for illustrative purposes only and is not limiting the scope of the invention as defined in the appended claims.

### Experimental section

### Methods

### Cohort of patients

A single-centre prospective observational study was conducted at the Bambino Gesù Paediatric Hospital in Rome between November 2022 and May 2023. The cohort comprised 122 predominantly paediatric patients with a diagnosis of epilepsy, cared for by the Neurology, Epilepsy and Movement Disorders Unit, and 29 healthy controls matched for age and sex, cared for by the Endocrinology Unit for suspected precocious puberty, subsequently excluded through diagnostic investigations. The controls were free of epilepsy or other neurological diseases. Patients undergoing experimental treatment, suffering from oncological or other progressive conditions, metabolic, inflammatory and infectious disorders were excluded, considering that microRNAs could be influenced by an underlying cause other than epilepsy itself. Subsequently, a longitudinal study was conducted on 47 of the previously analysed patients at t=0 who were re-analysed after 12 months under the same experimental conditions (t=1).

The study was conducted according to the ethical principles for medical research involving human participants stated in the Declaration of Helsinki and was approved by the local ethics committee (protocol no. 2858_OPBG_2022).

### Informed consent

After confirming eligibility through the inclusion and exclusion criteria, informed consents were obtained from the participants or both parents of each participant, if a minor or interdicted. The study used venous blood samples taken during regular clinical practice follow-up visits for both cases (at t=0 and at t=1) and controls, essentially integrating the research into normal blood investigations.

### Data Collection

For all participants, age at study enrolment and gender were collected. For cases, additional information was collected, including age at epilepsy onset, etiology of epilepsy, including genetic data and brain magnetic resonance imaging (MRI), seizure frequency, type of epilepsy (focal, generalised, combined), presence of comorbidities such as intellectual disability (ID) and/or behavioural problems, electroencephalogram (EEG) data, and ongoing ASM therapy. Seizures and epilepsies were classified according to the ILAE classification (Scheffer et al., 2017; Fisher et al., 2017).

This simplified approach facilitated a focused analysis of potential differences between the groups, ensuring that the objectives of the study were achieved with precision and relevance to the research questions posed.

### Sample collection

Blood (3-5 ml) was obtained using BD Vacutainer SST II Advance tubes (Becton Dickinson Italia S.p.A.). Standard venipuncture procedures were followed, which included the use of a 21-gauge needle to minimise haemolysis. Samples were allowed to clot for at least 30 minutes at room temperature. Subsequently, within 4 hours of collection, samples were centrifuged at room temperature (1300-2000 g for 10 minutes). The supernatant serum was removed, aliquoted and stored at - 80°C until use. Serum samples were anonymised and sent to the Genechron Srl laboratory (Rome) to be analysed for processing after blood sampling at the OPBG during the patients' periodicity. Frozen serum aliquots were thawed on ice prior to RNA extraction.

### Extraction of miRNA

Total RNA, including small RNA, was extracted from 200 µL of thawed serum using the miRNeasy Serum/Plasma Advanced kit (Qiagen, Germantown, MD; catalogue number 217204), according to the manufacturer's instructions. For normalisation, a non-human *Caenorhabditis elegans* cel-miR-39-3p oligonucleotide control was added to each serum sample in a fixed amount (1.5 ¹⁰ copies) prior to RNA extraction. Total RNA was eluted in 20 µL of nuclease-free water and stored at -80°C until further use.

### cDNA synthesis

The cDNA was prepared in 20 µL reactions using the Qiagen miRCURY LNA RT kit (3393404; Qiagen) according to the manufacturer's instructions and containing 4 µL of RNA. Reaction conditions for each 20 µL sample were as follows: reverse transcription step, 42 °C, 60 min; reaction inactivation at 95 °C, 5 min, maintenance 4 °C, using a Thermo Scientific 2720 thermal cycler. The cDNA (20 µL) was aliquoted to avoid repeated freezing and thawing cycles and stored at -20 °C until needed for qPCR.

### Quantification of miRNAs

Reverse transcription-qPCR (RT-qPCR) was performed using the miRCURY LNA SYBR Green PCR Kit (339347; Qiagen) and 6 miRCURY LNA miRNA PCR assays (Table A). One replicate for each sample was combined with each primer in triplicate on each 384-well plate. A 2-step cycle qPCR protocol was conducted (95 °C for 2 minutes followed by 40 cycles at 95 °C for 10 seconds and 56 °C for 60 seconds) using a Bio-Rad CFX384, Real-time C1000 Touch thermal cycler (Bio-Rad laboratories, ltd.). RT-qPCR involved no-template (NTC) and no-reverse transcriptase (NRTC) controls run with all 6 primers. Serum miRNAs were normalised and the Ct =2^{-ΔΔCt}method was applied.

**Table A. Description of the 6 miRCURY LNA miRNA PCR assays used for reverse transcription-qPCR**

| Primer assays for miRNA | Function of miRNAs |
|---|---|
| cel-miR-39-3p miRCURY LNA miRNA PCR Assay - 339306 | A non-human (Caenorhabditis elegans) and exogenous miRNA used for normalisation and evaluation of RNA and cDNA synthesis efficiency |
| hsa-miR-106b-5p miRCURY LNA miRNA PCR Assay - 339306 | analysed miRNA stably expressed |
| hsa-miR-146a-5p miRCURY LNA miRNA PCR Assay - 339306 | analysed miRNA stably expressed |
| hsa-miR-15a-5p miRCURY LNA miRNA PCR Assay - 339306 | analysed miRNA stably expressed |
| hsa-miR-451a miRCURY LNA miRNA PCR Assay - 339306 | stably expressed miRNA used for normalisation and detection of haemolysis |
| hsa-miR-23a-3p miRCURY LNA miRNA PCR Assay - 339306 | stably expressed miRNA used for normalisation and detection of haemolysis |

### Quality control and standardisation

Serum haemolysis was analysed with miR-23a-3p and miR-451a. MiR-23a-3p is stably present in serum/plasma and is not affected by haemolysis, whereas miR-451a is expressed in red blood cells. Samples with a delta Ct (miR-23a-miR-451) greater than 7 were considered to be affected by haemolysis. miR-23a-3p, miR-451a and cel-miR-39-3p spike-in were used for data normalisation.

### Data analysis and statistical analysis

The average Ct values and the variance of the triplicates performed for each sample were calculated for the 6 miRNA primers. Samples affected by haemolysis were identified (n=42). After normalisation, the Ct 2-ΔΔCt method was applied to assess the 'fold change' between cases and controls. The same method of analysis (Ct 2-ΔΔΔCt method) was applied to assess the 'fold change' between patients at t=0 and patients at t=1 (after 12 months).

The statistical methodology was designed to study the distribution and differential expression of the four miRNAs of interest in a cohort split between epilepsy patients and control subjects and subsequently between epilepsy patients at t=0 and t=1. This included a in-depth examination of miRNA levels in relation to the haemolysis status of the samples (haemolysed vs. non-haemolysed).

### Data normality and descriptive analysis

The initial steps involved assessing the normality of the data distribution for each of the 3 miRNAs tested (miR-106b-5p, miR-146a-5p, miR-15a-5p) using the Shapiro-Wilk test. Descriptive statistics were then calculated to provide a basic understanding of the distribution of data between all groups.

### Differential analysis of miRNAs

To identify differences in miRNA levels between epileptic patients and controls, the non-parametric Mann-Whitney test was used due to the non-normal distribution of the data, while for the differential analysis of miRNAs between the subgroups of patients (improved or stable-worsened) at t=0 and t=1, the Shapiro Wilk test was used due to the assumption of normality of the data (p>0.05). The use of the Mann-Whitney test in the analysis between patients and controls allowed the median miRNA values to be compared between the two groups, with statistical significance established at p<0.05 and a 95% confidence interval (CI).

### Comparison of haemolysis status

The analysis was extended to assess potential variations in miRNA expression related to the haemolysed state of the samples, but only when comparing cases and controls. Following a similar approach, the Shapiro-Wilk test was applied to assess the normality of the data, followed by the Mann-Whitney test to compare miRNA levels between haemolysed and non-haemolysed samples, according to the same significance criteria.

### Correlation with epilepsy-related variables

The study also aimed to explore correlations between miRNA expression and several epilepsy-related variables, including gender, type of epilepsy, aetiology, age at seizure onset, seizure frequency, type of epilepsy, ASM, ID and behavioural comorbidities. For this analysis, descriptive statistics were generated using contingency tables, focusing exclusively on the case group. Based on the normality test results, differences between miRNA levels and epilepsy-related factors were assessed using the Mann-Whitney and Kruskal-Wallis tests, as appropriate for the categorical variables at issue. The selection of these non-parametric tests is due to the non-normal distribution of miRNA data, with significance levels set at p-value < 0.05 and a 95% CI. Significantly, to further investigate the observed differences, post-hoc analyses were conducted following the Kruskal-Wallis tests. These analyses were essential to identify specific group comparisons driving the observed significant differences, thus providing a deeper understanding of the relationships between miRNA levels and specific epilepsy-related factors.

### Sub-cohort analysis

In a more detailed analysis, a sub-cohort consisting only of epilepsy patients and non-haemolysed samples was examined to discern any statistically significant differences in miRNA levels when considering epilepsy-related factors. This sub-cohort analysis was crucial to understand the specific impact of epilepsy, independent of haemolysis, on miRNA expression.

### Results

### Study population

The study enrolled 151 participants, classified into 122 epilepsy cases (56% male) and 29 healthy controls (31% male). Age matching between the groups was achieved, with a mean age of 8.3 years (range: 0.6-19.5 years) for the epilepsy group and 8.4 years (range: 2-15.3 years) for the controls. The results of the cohort characteristics are presented in Table 1.

The mean age of onset of epilepsy in the patients was 2.5 years (range: 0-14.6 years), with a mean disease duration of 5.8 years (range: 0.1-17.9 years), reflecting the chronic nature of the condition in the cohort.

Epileptic aetiologies were predominantly genetic (49.2%, n=60), followed by structural (28.7%, n=35) and unknown (22.1%, n=27). Clinical classification showed the majority of diagnoses to be focal epilepsy (64%, n=78), with fewer cases of generalised epilepsy (34%, n=42) and a minimal incidence of combined forms (1.6%, n=2).

The frequency of seizures varied significantly: 34.4% (n=42) of patients experienced sporadic seizures and 24.6% (n=30) were seizure free (SF), while others reported monthly (23%, n=28), daily (13.9%, n=17) or weekly (41%, n=5) episodes. The cohort was divided into two groups: the 'sporadic/SF' group, which includes patients who were SF or had sporadic seizures, and the 'other' group, which includes patients who experience monthly, weekly or daily seizures. Regarding treatment, almost half of the patients (48.4%, n=59) were on polypharmacy with ASM, 45.9% (n=56) received monotherapy and a small minority (5.7%, n=7) were not on ASM therapy. Valproic acid was the most frequently administered drug (n=48), followed by carbamazepine (n=39), levetiracetam (n=26) and clobazam (n=26).

EEG analysis indicated that more than half of the patients (54.1%, n=66) had epileptic abnormalities, while the rest had focal (37.7%, n=46) or generalised (8.2%, n=10) abnormalities. Brain MRI was normal in 41.8% (n=51) of cases, showed structural malformations underlying the epilepsy in 32% (n=39), showed non-specific abnormalities in 19.7% (n=24) and was not performed in 6.5% (n=8) of cases diagnosed with self-limiting epilepsy.

Cognitive assessments revealed that 45.1% (n=55) of the patients showed normal intellectual functioning. The remaining patients were classified into mild (20.5%, n=25), moderate (13.9%, n=17) or severe (20.5%, n=25) intellectual disability.

The psychiatric evaluation revealed that 23.7% (n = 29) had behavioural disorders (such as oppositional defiant disorder, attention-deficit/hyperactivity disorder, aggressive behaviour), 3.3% autism spectrum disorder (ASD) (n = 4) and 4.1% mood disorder (n = 5). Demographic and clinical data are shown in Table 1.

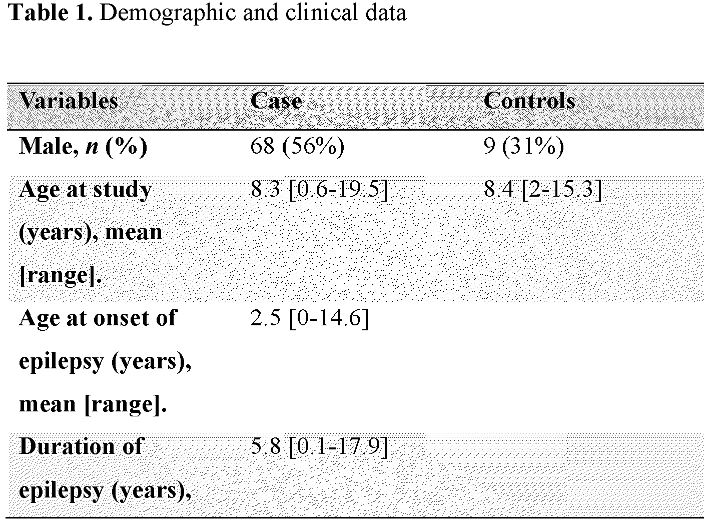

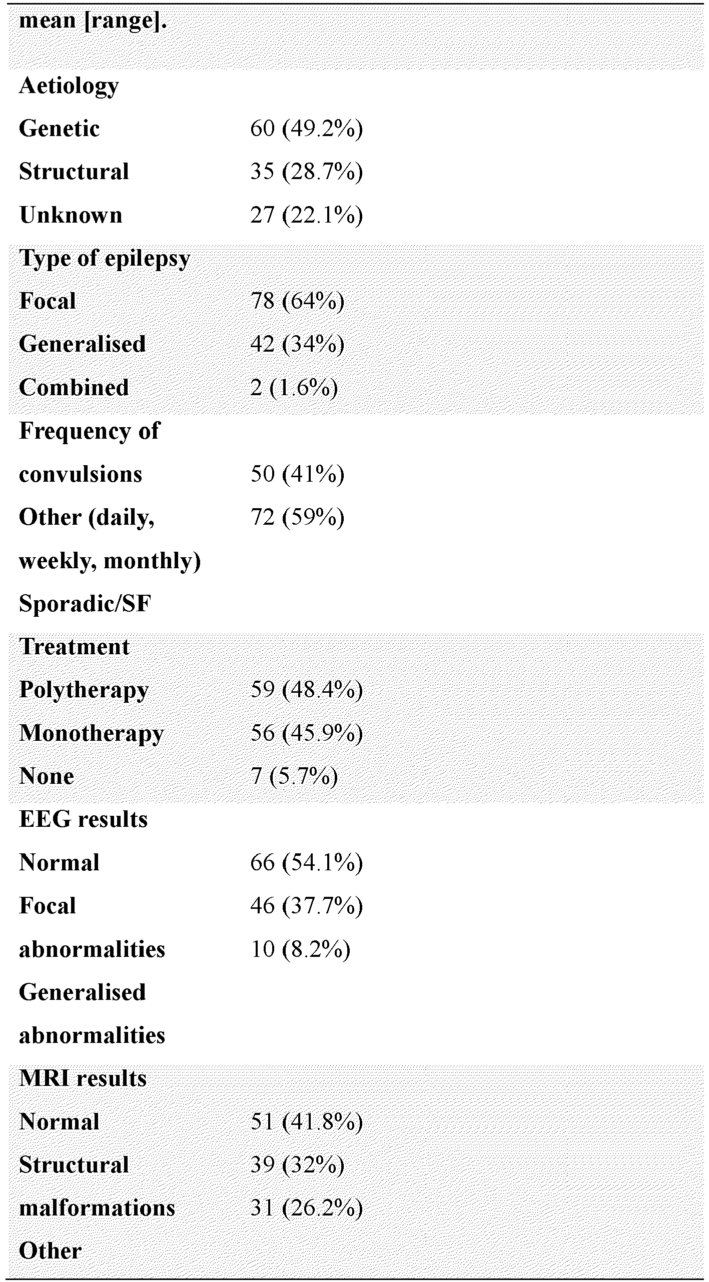

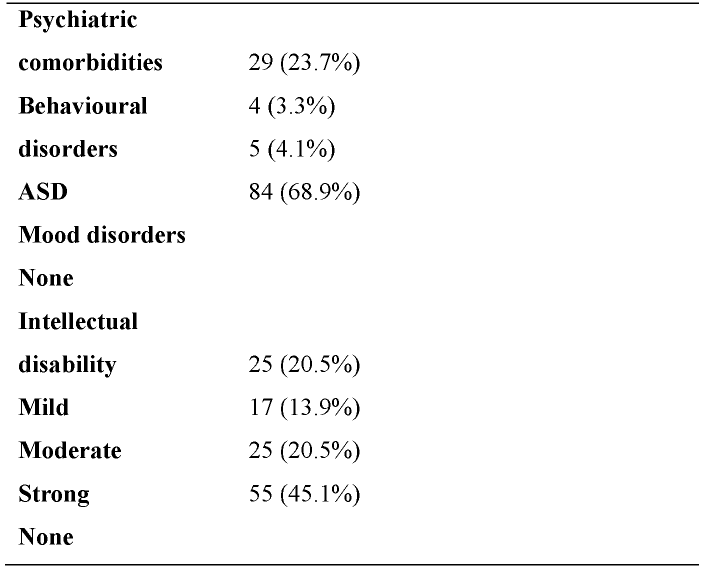

### Expression patterns of miRNAs

The study included 151 samples, 122 from patients with epilepsy (80.8%) and 29 from controls (19.2%). Haemolysed samples accounted for 27.8% (n=42) of the total and non-haemolysed samples were 72.2% (n=109) of the cohort. A longitudinal study was subsequently performed on 47 of the patients previously tested at t=0 and re-analysed under the same experimental conditions at t=1 (after 12 months).

In each of the following tables, the mean ± SD values correspond to the mean value of the natural logarithm of the RQ values for each miRNA, where RQ corresponds to Relative Quantification = 2^(-delta_Ct) while delta_Ct = Ct_mean_miRNA - Ct_mean_cel-miR-39-3p. The values reported, therefore, quantify the expression ratios in logarithmic mode. For this reason, higher absolute negative values correspond to lower miRNA expressions (sub-expressions).

Table 2 describes the miRNA analysis in the entire cohort and reveals a statistically significant difference in mean miRNA values between cases and controls. Specifically, miR-106b-5p, miR-146a-5p, miR-15a-5p all demonstrated statistically significant higher mean values in cases compared to controls, with p-values of 0.001, 0.001, 0.014 and 0.015, respectively, as determined by the Mann-Whitney (MW) test. This pattern suggests a potential role of these miRNAs in epilepsy.

Within the non-haemolysed samples, differences in miRNA levels remained statistically significant between cases and controls (p = 0.002, p = 0.000, p = 0.005 and p = 0.0025) as described in Table 2, reinforcing the potential diagnostic relevance of miRNA profiling in epilepsy. Notably, the 3 miRNAs were higher in cases than in controls.

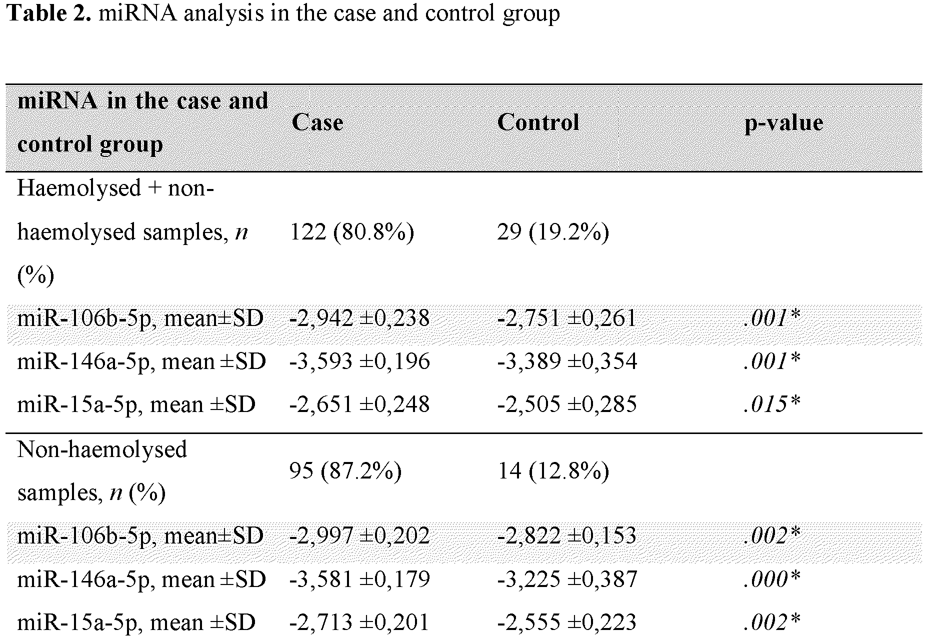

Further distinctions were observed between haemolysed and non-haemolysed samples in the case group, as shown in Table 3. In particular, the levels of miR-106b-5p and miR-15a-5p differed significantly between these two groups (p = 0.000), with lower levels in the haemolysed samples using the Mann-Whitney statistical test. No significant differences were found for miR-146a-5p (p=.062).

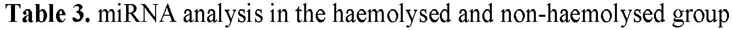

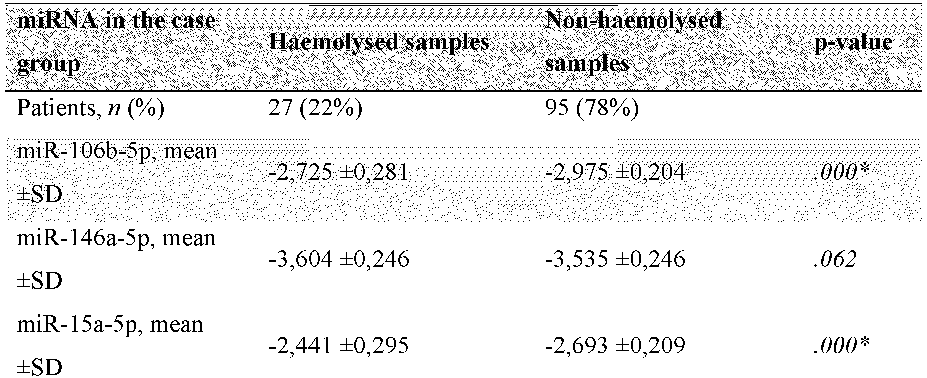

Furthermore, the study investigated the relationship between miRNA levels and various epilepsy-related variables such as sex, epilepsy type, aetiology, seizure frequency, ASMs, behavioural abnormalities and ID. No significant associations were found between miRNA levels and sex, ASMs, behavioural abnormalities or ID with p-value >0.05. However, significant variation in miRNA levels was observed according to seizure frequency (p = 0.017 and p = 0.004). Post hoc analysis showed that miR-106b-5p and miR-15a-5p levels showed statistically significant variation with seizure frequency (p = 0.017 and p = 0.004). The median value of both miR-106b-5p and miR-15a-5p was lower in the 'other' group (daily/weekly/monthly) than in the SF and sporadic group.

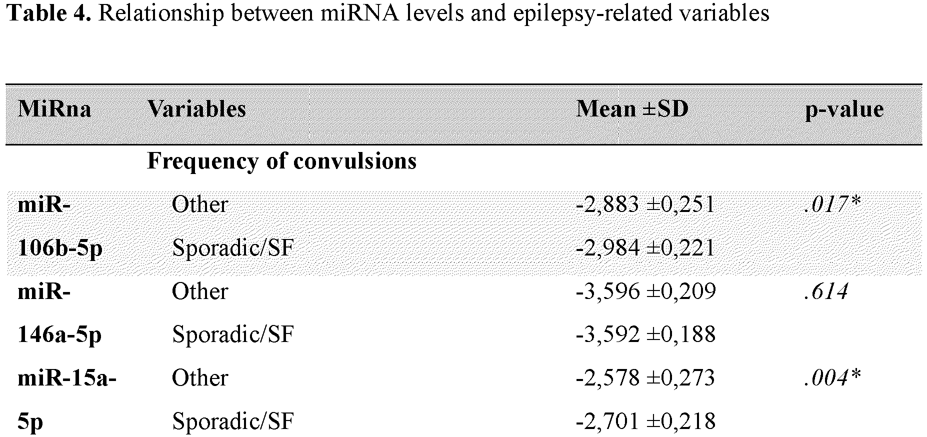

After further analysis of a sub-cohort including only cases and only non-haemolysed samples, no statistically significant differences were observed between miRNA values and all considered epilepsy factors (p>0.05). This analysis underlines the complexity of miRNA expression patterns and their interactions with clinical features of epilepsy.

While the first clinical validation showed a statistically significant variation in markers (in particular miR-106b-5p and miR-15a-5p) between patients and controls, the longitudinal test (carried out for these patients at t1 = t0 + 1 year) aimed to show whether a positive or negative change in the clinical situation of a certain patient would be accompanied by a correlated change in the values of the biomarkers miR-106b-5p and miR-15a-5p.

During the longitudinal study it was shown that miR-106b-5p and miR-15a-5p levels show statistically significant changes with improvement in seizure frequency. The median value of both miR-106b-5p and miR-15a-5p is lower in the 'stable-worse' group (daily/weekly/monthly) than in the 'improved' group (SF and sporadic).

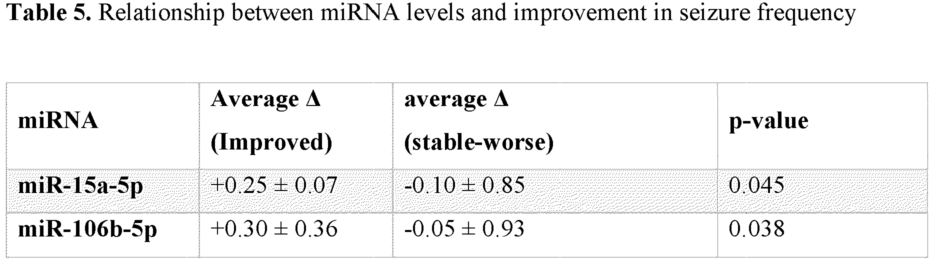

The results highlight the value of miRNAs as biomarkers in patients with epilepsy. In particular, miR-106b-5p and miR-15a-5p show the ability to distinguish between patients with different seizure frequencies, indicating their relevance in identifying individuals who do not respond to drugs or are at risk of developing drug resistance. Importantly, the stability of miRNAs in serum provides a non-invasive and accessible method to assess them, enabling routine clinical application.

### REFERENCES

An N, Zhao W, Liu Y, Yang X, Chen P Elevated serum miR-106b and miR-146a in patients with focal and generalized epilepsy. Epilepsy Res 127:311-316 (2016).
D. Organista-Ju'arez, A. Jim' enez, L. Rocha, M. Alonso-Vanegas, R. Guevara- Guzma'n, Differential expression of miR-34a, 451, 1260, 1275 and 1298 in the neocortex of patients with mesial temporal lobe epilepsy, Epilepsy Res. 157 (2019).
Sun J, Cheng W, Liu L, et al Identification of Serum miRNAs Differentially Expressed in Human Epilepsy at Seizure Onset and Post-Seizure. Mol Med Rep. (2016);14(6):5318.
Scheffer, I. E. et al. ILAE classification of the epilepsies: Position paper of the ILAE Commission for Classification and Terminology. Epilepsia 58, (2017).
Fisher, R. S. et al. The ILAE classification of seizures. Stanford Epilepsy Centre 58, (2017).

## Claims

1. An *in vitro* method for evaluating the epileptic condition of a patient, wherein the method comprises determining, in a serum sample from said patient, the level of a microRNA selected from hsa-miR-106b-5p and hsa-miR-15a-5p, said level being indicative of the frequency of the epileptic seizures of the patient.

2. The *in vitro* method according to claim 1, wherein the patient is under antiepileptic drug therapy and wherein said microRNA serum level is indicative of the response of the patient to the antiepileptic drug therapy.

3. The *in vitro* method according to claim 2, wherein a microRNA serum level below a reference value calculated from one or more therapy-respondent reference patients is indicative of non-responsiveness of the patient to the antiepileptic drug therapy.

4. The *in vitro* method according to claim 3, wherein said non-responsiveness is indicative of a drug resistance condition.

5. The *vitro* method according to any one of claims 1 to 4, wherein said microRNA is hsa-miR-106b-5p.

6. The *in vitro* method according to claim 5, wherein the hsa-miR-106b-5p serum level is determined by reverse transcription-qPCR.

7. The *in vitro* method according to any one of claims 1 to 4, wherein said microRNA is hsa-miR-15a-5p.

8. The *in vitro* method according to claim 7, wherein the hsa-miR-15a-5p serum level is determined by reverse transcription-qPCR.
